# FASCICULE DE BREVET EUROPEEN

(11) **EP 0 814 836 B1**
(45) Date de publication et mention de la délivrance du brevet: **14.03.2001**
(21) Numéro de dépôt: 95942007.6
(22) Date de dépôt: 21.12.1995
(51) Int. Cl.: A61K 39/39

(54) **ADJUVANTS POUR VACCINS COMPRENANT UN POLYSACCHARIDE SULFOLIPIDIQUE HYDROPHOBE**
EIN HYDROPHOBISCHES SULFOLIPIDISCHES POLYSACCHARID ENTHALTENDES ADJUVANS FÜR IMPFSTOFFE
ADJUVANT FOR VACCINES COMPRISING A HYDROPHOBIC SULFOLIPID POLYSACCHARIDE

(30) Priorité: 27.12.1994 BE 9401174
(43) Date de publication de la demande: 07.01.1998
(73) Titulaire: DIMMINACO AG, 8134 Adliswil (CH)
(72) Inventeur: HILGERS, Luuk, NL-3583 HE Utrecht (NL)
(74) Mandataire: Talbott, Dawn Jacqueline
(86) Numéro de dépôt international: BE9500119
(87) Numéro de publication internationale: WO9620008

(56) Documents cités:
- EP-A- 0 549 074
- JAPAN JOURNAL OF EXPERIMENTAL MEDICINE, vol. 58, no. 3, pages 145-151, MIZUMOTO K. ET AL. 'Sulfated Homopolysaccharides with Immunomodulating Activities are More Potent Anti-HTLV-III Agents than Sulfated Heteropolysaccharides'
- IMMUNOLOGY, vol. 60, no. 1, OXFORD, pages 141-146, HILGERS L.A.T., ET AL 'Synthetic sulphopolysaccharides: novel adjuvants for humoral immune responses'
- VACCINE, vol. 12, no. 7, GUILDFORD GB, pages 653-660, HILGERS L., ET AL. 'A novel non-mineral oil-based adjuvant. I.' cited in the application

## Description

La présente invention a pour objet de nouveaux adjuvants pour vaccins.

Un antigène est défini comme étant une substance étrangère à un organisme vivant, qui lorsqu'on l'administre, par exemple, par voie parentérale induit une réponse immunitaire, par exemple, la formation d'anticorps.

Les anticorps sont des substances contenues dans le sang et autres fluides du corps, ainsi que dans les tissus, qui se lient à l'antigène pour le rendre inoffensif. Les anticorps constituent un des mécanismes naturels de défense du corps. Ils sont hautement spécifiques et peuvent tuer, lier ou rendre inoffensif l'antigène qui a induit leur formation.

L'antigène, en contact avec le système immunitaire. active donc une série complexe d'interactions cellulaires dont le but est d'éliminer l'antigène et/ou de rétablir l'équilibre précédent.

Deux des aspects caractéristiques des antigènes sont leur immunogénicité, c'est-à-dire leur capacité d'induire une réponse immunitaire in vivo (entre autres la formation d'anticorps spécifiques) et leur antigénicité, c'est-à-dire leur capacité d'être reconnu sélectivement par les anticorps dont les antigènes sont à l'origine.

Il est connu de stimuler la réponse immunitaire délibérément en administrant un antigène spécifique au moyen d'un vaccin. Cette procédure permet d'obtenir un état de mémoire immunitaire dans l'organisme qui permet une réponse plus rapide et plus efficace de l'organisme lors d'un contact ultérieur avec l'antigène.

Cependant, quelques antigènes ne possèdent qu'une faible immunogénicité et induisent une réponse immunologique insuffisante pour procurer à l'organisme une protection efficace.

L'immunogénicité d'un antigène peut être augmentée en l'administrant ensemble avec des substances, appelées adjuvants, qui augmentent la réponse contre l'antigène soit en agissant directement sur le système immunitaire soit en modifiant les caractéristiques pharmacocinétiques de l'antigène et en augmentant ainsi le temps d'interaction avec le système immunitaire.

A présent, un grand nombre de vaccins vétérinaires utilisés en tant qu'adjuvants comprennent encre les émulsions classiques d'huile minérale, tels que l'adjuvant de type eau dans huile minérale (w/o) ou de type huile minérale dans eau (o/w). Depuis plusieurs années, des recherches ont été entreprises pour trouver des alternatives ayant une efficacité similaire mais une toxicité réduite. Les injections de ces adjuvants classiques à base d'huile minérale sont souvent accompagnées par des réactions locales dont la sévérité dépend largement du type d'émulsion et de la nature de l'huile utilisée. L'utilisation d'adjuvants à base d'huile minérale est par conséquent limitée aux animaux domestiques (porcs, poules, ruminants, etc.) et aux animaux de laboratoire.

Le document EP-A-0 549 074 décrit l'utilisation de lipopolysaccharides synthétiques comme adjuvants.

Précédemment, il a été montré qu'un copolymère synthétique de polysucrose et épichloridrine - le Ficoll - portant des groupes sulfate et lipides (SL-Ficoll), incorporé dans une émulsion de squalane (S) dans de l'eau (S/W) exerçait un effet adjuvant élevé sur différentes espèces d'animaux - dont des porcs - contre différents types d'antigènes, y compris quelques antigènes viraux importants [Vaccine 12, p. 653-660 (1994) et Vaccine 12, p. 661-665 (1994), EP-A-0 549 074]. Ces formulations d'adjuvants à base de Ficoll sont assez efficaces pour remplacer les formulations classiques d'huile minérale dans l'eau utilisées dans différents vaccins porcins.

Cependant la toxicité locale, c'est-à-dire la réactogénicité, de ces formulations à base de Ficoll sur des porcs et des souris ne s'est pas révélée plus faible que celle des formulations classiques d'huile minérale de type o/w.

De plus, pour les formulations à base de Ficoll, la température a un effet prononcé sur la stabilité des émulsions. Certaines de ces émulsions ont été stables pendant des années à 4°C mais la phase aqueuse et la phase d'huile se séparent endéans quelques jours à 37°C et endéans une dizaine de minutes à 60°C.

Le but de la présente invention est de proposer un adjuvant efficace pour vaccins ayant une stabilité accrue à haute température et révélant une toxicité locale plus faible.

Ce but est atteint par un adjuvant pour vaccins comprenant un polysaccharide sulfo-lipidique combiné à un composant formant une interface [p. ex., une émulsion de type huile/eau (o/w)].

Un des avantages de l'adjuvant selon la présente invention est qu'il est plus stable à haute température que les adjuvants à base de Ficoll.

Ce but est atteint par un adjuvant pour vaccins, comprenant un polysaccharide sulfolipidique hydrophobe combiné à un constituant formant une interface, dans lequel le polysaccharide est choisi dans le groupe consistant en cyclodextrine, maltodextrine, inuline et pullulane, les groupes lipidiques comprennent 4 à 22 atomes de carbone et le polysaccharide sulfolipidique contient en moyenne, au moins 0,01 et pas plus de 1,0 groupe sulfate par monosaccharide, en moyenne au moins 0,01 et par plus de 2,0 groupes lipidiques par monosaccharide, et le rapport des groupes sulfate aux groupes lipidiques est un rapport de 0,01 à 2 groupes sulfate par groupe lipidique, tout en maintenant son caractère hydrophobe.

L'expression "polysaccharide" signifie un composé ayant au moins trois unités sucres répétitives reliées l'une à l'autre de manière covalente.

L'expression "polysaccharide sulfo-lipidique" signifie un composé ayant au moins trois unités répétitives reliées l'une à l'autre de manière covalente, au moins un groupe de sulfate et au moins un groupe lipidique.

Le polysaccharide sulfo-lipidique est un polysaccharide hydrophobe.

L'expression "polysaccharide hydrophobe" signifie un polysaccharide qui est moins soluble dans une phase aqueuse que dans une phase organique apolaire.

Le polysaccharide sulfo-lipidique est choisi parmi le groupe constituét par les cyclodextrines, la maltodextrine, l'inuline et le pullulane.

De préférence, le polysaccharide sulfo-lipidique est choisi parmi le groupe constitué par la cyclodextrine, la maltodextrine et l'inuline.

Le polysaccharide sulfo-lipidique préféré est la cyclodextrine.

Le polysaccharide sulfo-lipidique contient en moyenne au moins 0,01 groupe sulfate par monosaccharide, toute en maintenant son caractère hydrophobe. De préférence, le polysaccharide sulfo-lipidique contient en moyenne au moins 0,12 groupe slfate par monosaccharide, tout en maintenant son caractère hydrophobe.

Le polysaccharide sulfo-lipidique ne contient en moyenne pas plus de 1,0 groupe sulfate par monosaccharide, tout en maintenant son caractère hydrophobe. De préférence, le polysaccharide sulfo-lipidique ne contient en moyenne pas plus de 0,23 groupe sulfate par monosaccharide, tout en maintenant son caractère hydrophobe.

De préférence, quand le polysaccharide sulfo-lipidique est de la maltodextrine, celui-ci contient en moyenne environ 0,23 groupe sulfate par monosaccharide, toute en maintenant son caractère hydrophobe.

De préférence, quand le polysaccharide sulfo-lipidique est de la cyclodextrine, celui-ci contient en moyenne environ 0,20 groupe sulfate par monosaccharide, tout en maintenant son caractère hydrophobe.

De préférence, quand le polysaccharide sulfo-lipidique est de l'inuline, celui-ci contient en moyenne environ 0,19 groupe sulfate par monosaccharide, tout en maintenant son caractère hydrophobe.

De préférence, quand le polysaccharide sulfo-lipidique est du pullulane, celui-ci contient en moyenne environ 0.16 groupe sulfate par monosaccharide, tout en maintenant son caractère hydrophobe.

Le polysaccharide sulfo-lipidique contient en moyenne au moins 0,01 groupe lipidique par monosaccharide, tout en maintenant son caractère hydrophobe. De préférence, le polysaccharide sulfo-lipidique contient en moyenne au moins 1,05 groupe lipidique par monosaccharide, tout en maintenant son caractère hydrophobe.

Le polysaccharide sulfo-lipidique ne contient en moyenne pas plus de 2,0 groupes lipidiques par monosaccharide, tout en maintenant son caractère hydrophobe. De préférence, le polysaccharide sulfo-lipidique ne contient en moyenne pas plus de 1,29 groupes lipidiques par monosaccharide, tout en maintenant son caractère hydrophobe.

De préférence, quand le polysaccharide sulfo-lipidique est de la maltodextrine, celui-ci contient en moyenne environ 1,29 groupes lipidiques par monosaccharide, tout en maintenant son caractère hydrophobe.

De préférence, quand le polysaccharide sulfo-lipidique est de la cyclodextrine, celui-ci contient en moyenne environ 1,05 groupes lipidiques par monosaccharide, tout en maintenant son caractère hydrophobe.

De préférence, quand le polysaccharide sulfo-lipidique est de l'inuline, celui-ci contient en moyenne environ 1,24 groupes lipidiques par monosaccharide, tout en maintenant son caractère hydrophobe.

De préférence, quand le polysaccharide sulfo-lipidique est du pullulane, celui-ci contient en moyenne environ 1.24 groupes lipidiques par monosaccharide, tout en maintenant son caractère hydrophobe.

Les groupes lipidiques contiennent, entre 4 et 22 atomes de carbone.

Le rapport des groupes sulfates et lipidiques est compris entre 0,01 et 2 groupes sulfates par groupe lipidique. Le rapport des groupes sulfates et lipidiques est compris entre 0,10 et 0,19 groupe sulfate par groupe lipidique, tout en maintenant le caractère hydrophobe du composé.

De préférence, quand le polysaccharide sulfo-lipidique est de la maltodextrine, le rapport des groupes sulfates et lipidiques est d'environ 0,18 groupe sulfate par groupe lipidique, tout en maintenant son caractère hydrophobe.

De préférence, quand le polysaccharide sulfo-lipidique est de la cyclodextrine, le rapport des groupes sulfates et lipidiques est d'environ 0,19 groupe sulfate par groupe lipidique, tout en maintenant son caractère hydrophobe.

De préférence, quand le polysaccharide sulfo-lipidique est de l'inuline, le rapport des groupes sulfates et lipidiques est d'environ 0,15 groupe sulfate par groupe lipidique, tout en maintenant son caractère hydrophobe.

De préférence, quand le polysaccharide sulfo-lipidique est du pullulane, le rapport des groupes sulfates et lipidiques est d'environ 0,13 groupe sulfate par groupe lipidique, tout en maintenant son caractère hydrophobe.

L'expression "composant formant une interface" ("Interface-Forming Constituent" ou "IFC") signifie un substance qui forme, dans un milieu aqueuse, une interface physique entre la substance et la phase aqueuse.

Le composant formant une interface est choisi parmi le groupe constitué d'un liquide non miscible à l'eau (par exemples : squalane, huile de soja, huile minérale, hexadécane), ou un solide insoluble dans la phase aqueuse.

Les solides insoluble dans la phase aqueuse de la présente invention comprennent des sels insolubles (p. ex. Al(OH)₃, AlPO₄, Alun, oxalate de calcium). des microparticules, des nanoparticules, des microsphères et des nanosphères d'un ou des multiples polymères, copolymères (p.ex. polyacrylate, poly(methyl methacrylate), polycyanoacrylate, polylactide, polyglycolide), ou des bi-couches lipidiques ou des agents lipophiles (p.ex., phospholipides) ou des micelles d'agents tension-actifs.

De préférence, le composant "IFC" est un liquide non miscible à l'eau.

Avantageusement, le composant formant une interface est choisi parmi le groupe constitué d'huile de soja, de squalane et hexadécane.

Les adjuvant stables pour vaccins sont ceux qui comprennent un dérivé (hydrophobe) d'un polysaccharide sulfo-lipidique, un composant formant une interface et un agent émulsifiant.

De plus, l'efficacité des adjuvants selon la présente invention est comparable ou même supérieure à celle des adjuvants classiques. La toxicité locale, c'est-à-dire la réactogénicité des adjuvants selon la présente invention est, en général, inférieure à celle des adjuvants classiques et à celle des adjuvants à base de ficoll.

Il semblerait que les polysaccharide sulfo-lipidique à poids moléculaires relativement plus faibles aient une moindre toxicité locale.

Selon un autre aspect de la présente invention, on propose l'utilisation du polysaccharide sulfo-lipidique en tant qu'adjuvant dans des vaccins.

Selon encore un autre aspect de la présente invention, on propose un procédé pour préparer un vaccin en émulsion caractérisé en ce que l'on émulsionne une solution aqueuse d'un antigene en présence d'un polysaccharide sulfo-lipidique, d'un émulsifiant et un composant formant une interface.

Selon un autre aspect de la présente invention, on propose un vaccin comprenant une quantité immunogénique d'un antigène (immunogène) et un adjuvant selon la présente invention.

De préférence, la concentration d'adjuvants est comprise entre 0.1 et 100 mg/ml, de préférence entre 2 et 20 mg/ml.

Le vaccin comprend, outre l'adjuvant, des antigènes par exemple de virus inactivé, de virus vivant, de bactérie, de sous-unité, de protéine, de peptide et de virus influenza inactivé souche MRC-11, d'ovalbumine (OVA), de virus influenza inactivé souche A/Swine et/ou de virus de pseudorabies inactivés (iPRV).

Les titres en anticorps mesurés ont été plus élevés que ceux obtenus par les émulsions d'huile minérale dans de l'eau utilisées dans des produits commerciaux. Il a été montré qu'il existe une forte activité synergique entre les SL-Ficoll et les émulsions, plus prononcée sur les porcs que sur les souris (Hilgers et al. Vaccine 12, 661-664. 1994). La réponse d'anticorps sur les souris a été augmentée d'une manière significative (Hilgers et al. Vaccine 12, 653-660, 1994).

On a conclu que la réactogénicité dépend du type de polysaccharide et d'huile inclus dans les formulations et que le poids moléculaire du polysaccharide est un des facteurs importants.

Les Cyclodextrines et leurs dérivés sont bien connus pour être capables de former des complexes d'inclusion en abritant d'autres substances, comme p.ex. des substances d'intérêt pharmaceutique, dans la cavité formée par leur structure cyclique.

Parmi les avantages offerts par ces complexes d'inclusion, on peut citer l'amélioration de la solubilité, de la biodisponibilité et/ou de la stabilité chimique, l'allongement du temps de demi-vie, la diminution des effets secondaires ainsi que certains avantages lors de la production tels qu'une obtention plus aisée de poudre sèche au départ de préparations liquides.

Les Polysaccharides Sulfo-Lipidique (SLP) à base de Cyclodextrine peuvent dès lors avoir des applications intéressantes en plus de leur utilisation comme adjuvant décrite ci-dessus.

En effet, les SL-Cyclodextrines sont des agents tensio-actifs par suite de la présence simultanée dans ces molécules de groupes anioniques (sulfates) et hydrophobes (chaînes aliphatiques). Cette propriété est à l'origine de la formation de micelles en phase aqueuse ou de micelles mixtes en présence d'autres substances tensio-actives, ainsi que de la formation d'émulsions de liquides non miscibles dans l'eau, de suspensions en phase aqueuse de particules insolubles ou de la formation d'interfaces entre phase aqueuse et matière insoluble liquide ou solide.

Ainsi, les SL-Cyclodextrines présentent le double avantage de pouvoir former des complexes d'inclusion et d'être des substances tensio-actives.

Elles peuvent donc être considérées comme formant une famille de produits présentant des propriétés nouvelles susceptibles d'applications originales dans le secteur pharmaceutique.

De nombreuses SL-Cyclodextrines différentes ont été obtenues; elles se distinctent par leurs propriétés physico-chimiques résultant du type de Cyclodextrine (p.ex,α,β,γ) de la teneur en groupes sulfates ainsi que de la teneur et de la nature des groupes lipidiques.

### Exemple 1

Différents polysaccharides ont été testés pour leur solubilité dans les solvants organiques utilisés pour la synthèse et ceux insuffisamment solubles ont été abandonnés. 4,5 grammes de la maltodextrine (Maltodextrine15; Amylum, Belgium), 4,5 grammes de la béta-cyclodextrine (ACROS), 4,5 grammes de l'inuline (Dahlia Tubers; Sigma, U.S.A.) et 4,5 grammes du pullulane (Hayashibara Co. Ltd. Japon) ont été solubilisés séparément dans des mélanges (100 ml) de diméthylformamide anhydre et de pyridine anhydre (rapport volumique 1:1). Pendant une courte période de temps (48 heures), les solutions de ces polysaccharides ont été séchées à l'aide de tamis moléculaires (size 2Å) (Merck, Darmstadt, Allemagne). 6,6 grammes du chlorure de lauroyle (Merck, Germany) a été ajouté et les mélanges ont été incubés pendant 6 heures à 60 °C et pendant 18 heures à température ambiante (environ 15 °C - 22 °C). Ensuite, 0,6 grammes de l'acide chlorosulfonique (Merck, Germany) dans un mélange (10 ml) de diméthylformamide anhydre et de pyridine anhydre a été ajouté et la procédure d'incubation à température ambiante a été renouvelée. Les solvants ont été éliminés par évaporation à pression réduite (200 millibars) pour 1-2 heures à 60 °C et dialysés en utilisant une membrane de cellulose régénérée ayant un seuil de coupure de 10.000 Daltons (SPECTRA/POR) en mettant en oeuvre une solution saline tamponnée de phosphate isotonique (cette solution saline PBS comprend, par litre d'eau, 8 g de chlorure de sodium, 0,2 g de chlorure de potassium et 1,15 g de phosphate hydrogéné disodique ayant un pH de 7,3) et postérieurement en mettant en oeuvre de l'eau ultrapure jusqu'à ce qu'aucune trace de solvent ne soit détectée dans le filtrat. Le rapport volumique entre la solution de dialyse (solution saline PBS ou eau ultrapure) et le reliquat utilisé au cours de la dialyse est maintenu au dessus de 10:1 (v/v). La dialyse est réalisée durant au moins 10 jours, la solution de dialyse étant remplacée au moins une fois par jour. Les polysaccharides sulfo-lipidiques (SLP) ainsi obtenus ont été lyophilisés à température ambiante, avec une pression interne inférieure à 0,1 mbar et un piège à froid ayant une température inférieure à -25 °C.

La composition des SLP a été déterminée en dosant le polysaccharide, les sulfates liés, le contenu total en lipides et le contenu en lipides liés tel que décrit précédemment (Vaccine 12, p. 653-660).

Des solutions de 1 % poids/volume (p/v) de SLP dans une solution saline tamponnée au phosphate (PBS) contenant 2 % volume/volume (v/v) de Tween® 80 ont été préparées en dissolvant d'abord les SLP dans un volume minimal (20 ml) de méthyltertio-butyléther (Merck), en ajoutant 2 ml de Tween ® 80 (Merck) par g de SLP et en évaporant le méthyltertio-butyléther à température élevée (± 60 °C) et à pression réduite (50 millibars) jusqu'à ce qu'une solution visqueuse de SLP dans du Tween 80 ait été obtenue. Dix ml d'eau par g de SLP ont été ajoutés lentement et ensuite les volumes appropriés de PBS et d'huile ont été ajoutés. Le mélange a été émulsifié en utilisant un microfluidiseur (Microfluidics Corp., Newton, U.S.A) jusqu'à ce que plus aucune goutte d'huile supérieure à 2 ou 3 µm ne soit visible sous le microscope (agrandissement de 1000 fois). Les émulsions ont été stockées à 4 °C jusqu'à utilisation.

Les vaccins ont été préparés en mélangeant un volume d'antigène avec un volume d'adjuvant. Les antigènes ont été préparés tel que décrit précédemment (Vaccine 12, p. 653-660 et Vaccine 12, p. 661-665). Deux solutions d'antigènes différentes ont été utilisées : la solution I comprend par ml : 10 µg de virus influenza inactivé souche MRC-11 (SOLVAY DUPHAR) + 1000 µg OVA (SIGMA) et la solution II comprend par ml : 4,4 µg/ml de virus influenza inactivé souche A/Swine (SOLVAY DUPHAR), 4,0 µg de virus influenza inactivé de la souche MRC-11 (SOLVAY DUPHAR), 2,0 µg de virus influenza inactivé de la souche X-79 (SOLVAY DUPHAR), et 10⁸ TCID₅₀ de virus de pseudorabies inactivés (iPRV) (SOLVAY DUPHAR).

Des souris femelles NMRI (Charles River, Germany) de 8 à 10 semaines (environ 20 g) ont été immunisées par injection dans la patte avec 0,025 ml de vaccin et les titres en anticorps ont été mesurés trois semaines plus tard.

Les titres en anticorps ont été exprimés en tant que moyennes géométriques (²log + SEM). L'analyse des titres d'anticorps a été effectuée par des tests standardisés et les critères pour la validité ont été décrits précédemment (Vaccine 12, p. 653-660).

Des tests de Student ont été effectués pour analyser la signification statistique des résultats et une P < 0,05 a été considérée comme étant significative.

Des formulations d'adjuvants ont été testées dans deux expériences indépendantes sur des souris.

Tableau 1: L'effet adjuvant de certaines émulsions à base de polysaccharides sulfo-lipidiques (SLP) dans de l'huile et de l'eau (SLP/o/w) sur des souris.

| Adjuvant [mg SLP et µl huile]/ml | n | ²log du titre d'anticorps contre | | | |
|---|---|---|---|---|---|
| | | MRC 11 | | OVA | |
| | | moyenne | SEM | moyenne | SEM |
| Expérience I | | | | | |
| SL-Maldexl5/S/W [10/100] | 6 | 13.1 | 0.7 | 7.3 | 0.2 |
| SL-Cyclodex./S/W [10/100] | 6 | 12.9 | 1.0 | 7.2 | 0.6 |
| SL-Inuline/S/W [10/100] | 6 | 12.7 | 0.8 | 7.2 | 0.2 |
| SL-Pullulane/S/W [10/100] | 6 | 13.3 | 0.7 | 6.9 | 0.4 |
| SL-Maldexl5/soja/W [10/100] | 6 | 12.8 | 0.6 | 6.5 | 0.7 |
| SL-Cyclodex./soja/W [10/100] | 6 | 13.6 | 1.1 | 6.0 | 0.8 |
| SL-Inuline/soja/W [10/100] | 6 | 12.5 | 0.6 | 6.8 | 1.3 |
| SL-Pullulane/soja/W [10/100] | 6 | 12.5 | 0.8 | 6.6 | 0.4 |
| SL-Ficoll/S/W [10/100] | 6 | 13.0 | 0.9 | 6.9 | 0.4 |
| SL-Ficoll/soja/W [10/100] | 6 | 12.0 | 0.6 | 6.6 | 0.3 |
| SL-Ficoll/hexadéc./W [10/100] | 4 | 12.4 | 0.9 | 7.2 | 0.6 |
| SL-Ficoll/h. minérale/W [10/100] | 4 | 12.2 | 0.5 | 6.8 | 0.3 |
| Contrôle (sans adjuvant) | 6 | 11.3 | 0.7 | 3.5 | 2.0 |

| Expérience II | | | | | |
|---|---|---|---|---|---|
| SL-Maldexl5/S/W [10/100] | 6 | 13.3 | 0.8 | 9.0 | 1.1 |
| SL-Cyclodex./S/W [10/100] | 6 | 13.0 | 0.6 | 8.6 | 0.8 |
| SL-Inuline/S/W [10/100] | 6 | 12.8 | 0.9 | 8.6 | 0.8 |
| SL-Pullulane/S/W [10/100] | 6 | 13.6 | 0.5 | 8.8 | 0.8 |
| SL-Maldex15/soja/W [10/100] | 6 | 11.2 | 0.7 | 7.4 | 0.3 |
| SL-Cyclodex./soja/W [10/100] | 6 | 11.7 | 0.5 | 6.5 | 1.0 |
| SL-Inuline/soja/W [10/100] | 6 | 11.8 | 0.5 | 7.5 | 0.4 |
| SL-Pullulane/soja/W [10/100] | 6 | 10.9 | 0.8 | 7.3 | 0.4 |
| SL-Maldex15/hexadéc./W [10/100] | 6 | 12.2 | 0.7 | 8.3 | 0.8 |
| SL-Cyclodex./hexadéc/W [10/100] | 6 | 12.5 | 0.6 | 8.2 | 0.6 |
| SL-Inuline/hexadéc./W [10/100] | 6 | 12.2 | 0.4 | 7.4 | 0.4 |
| SL-Pullulane/hexadéc/W [10/100] | 6 | 13.2 | 0.4 | 7.7 | 0.2 |
| Contrôle (sans adjuvant) | 6 | 10.0 | 0.4 | 4.2 | 0.9 |
| SL-Ficoll/S/W [10/100] | 2 | 13.4 | 0.7 | 8.5 | 0.7 |
| n = nombre d'animaux soja = huile de soja | | | | | |
| SEM = déviation standard de la moyenne hexadéc. = hexadécane | | | | | |
| Maldex15 = maltodextrine15 h. minérale = huile minérale | | | | | |
| Cyclodex. = cyclodextrine S = squalane | | | | | |
| W = eau (water) | | | | | |

Toutes les émulsions SLP/huile/eau ont augmenté la réponse humorale contre le virus de l'influenza souche MRC-11 et contre OVA. Il a été montré qu'il existe des différences en effet adjuvant entre les différentes formulations et quelques-unes ont provoqué des activités égales au SL-Ficoll/S/W. Aucun effet clair dû au type de SLP n'a pu être observé. En ce qui concerne le type d'huile utilisé, des émulsions comprenant du squalane (S) ont donné des réponses significativement plus élevées que les émulsions contenant de l'hexadécane tandis que l'huile de soja provoquait les réponses les moins élevées.

### Exemple 2

Des porcs de 8 à 10 semaines d'âge ont été testés pour constater une éventuelle présence d'anticorps contre les antigènes viraux en question et les animaux avec des titres d'anticorps détectables ont été exclus.

Une émulsion minérale huile dans l'eau vendue sous la marque SUVAXYN O/W EMULSION (SOLVAY DUPHAR) ainsi que des formulations de SLP dans une émulsion de squalane dans l'eau ont été préparées selon la méthode décrite à l'exemple 1.

Des vaccins contenant les formulations avec adjuvant ont été préparés selon la méthode décrite à l'exemple 1.

Des formulations de SLP/squalane/eau ont été testées pour leur effet adjuvant sur des porcs avec l'iPRV et le Virus Influenza Inactivé - souches MRC-11 et A/Swine, en tant qu'antigènes (Tableau 2). Les animaux ont été immunisés deux fois (semaines 0 et 3) et les titres en anticorps ont été mesurés trois semaines (semaine 6) après la deuxième injection.

Les titres en anticorps contre PRV (anti-PRV) ont été augmentés par les émulsions SL-Ficoll/squalane/eau, SL-Maltodextrine15/squalane/eau, SL-Inuline/squalane/eau et SL-Cyclodextrine/squalane/eau. Les titres en anticorps mesurés ont été comparables ou plus élevés que ceux obtenus par les émulsions d'huile minérale dans de l'eau utilisées dans des produits commerciaux. La réponse d'anticorps contre A/Swine a aussi été augmentée par différentes formulations SLP/squalane/eau mais certaines réponses ont été aussi élevées que celles obtenues par les émulsions huile/eau classiques. Les titres en anticorps contre MRC-11 ont été augmentés par différentes formulations et quelques émulsions SLP/squalane/eau ont montré des titres comparables ou plus élevés que les émulsions huile minérale/eau.

Les résultats de ces analyses sont repris dans le tableau 2.

Tableau 2 : Effets de différents polysaccharides sulfo-lipidiques combinés avec plusieurs émulsions d'huile dans de l'eau sur la réponse d'anticorps anti-iPRV/A/swine/MRC-11 mesurés sur des porcs.

| Adjuvant [mg SLP+µl huile]/ml | ²log des titres d'anticorps à 6 semaines | | | | | |
|---|---|---|---|---|---|---|
| | iPRV | | A/Swine | | MRC-11 | |
| | moyen. | SEM | moyen. | SEM | moyen. | SEM |
| PBS | 0.5 | 0.0 | <3.3 | 0.0 | <3.9 | 0.5 |
| huile minérale/W [0/500] | 6.5 | 0.4 | 10.7 | 1.3 | 10.7 | 0.9 |
| SL-Ficoll/S/W [10/100] | 7.8 | 0.8 | 11.5 | 2.9 | > 12.7 | 2.4 |
| SL-Maldexl5/S/W 10/100] | 7.5 | 1.1 | 8.7 | 1.1 | 13.1 | 1.3 |
| SL-Cyclodex./S/W [10/100] | 9.0 | 1.1 | 10.1 | 1.5 | >12.9 | 2.2 |
| SL-Inuline/S/W [10/100] | 8.9 | 1.3 | 10.3 | 1.0 | >13.7 | 1.3 |
| SEM = déviation standard de la moyenne | | | | | | |
| S = squalane | | | | | | |
| Maldex15 = maltodextrine15 | | | | | | |
| W = eau (water) | | | | | | |
| 20 Cyclodex. = cyclodextrine | | | | | | |

A partir d'expériences précédentes pour tester l'effet adjuvant en parallèle sur ces deux espèces d'animaux, il a été montré qu'il existe une forte activité synergique entre les SL-Ficolls et les émulsions et qu'elle est plus prononcée sur les porcs que sur les souris (Vaccine 12, 653-660, 1994; Vaccine 12, 661-664, 1994). La réponse d'anticorps sur les souris a été augmentée d'une manière significative et il a été montré qu'il existe un effet prononcé du type d'huile employé mais non pas du type de polysaccharide.

Le facteur d'augmentation en comparaison avec des animaux qui n'ont reçu que l'antigène a été limité à une ou deux unités ²log sur les souris. Sur les porcs, l'effet adjuvant a été plus prononcé principalement parce que la réponse contre les antigènes sans adjuvant a été très faible. Les titres en anticorps anti-iPRV et anti-A/wine et anti-MRC-11 ont été augmentés de plus de six unités ²log.

Les adjuvants à base d'huile minérale augmentent la réponse d'anticorps d'une manière significative, mais différentes nouvelles formulations d'adjuvants ont provoqué des titres encore plus élevés. L'adjuvant SL-Ficoll/S/W présenté précédemment (Vaccine 12, p. 653-660 et Vaccine 12, p. 661-665) s'est révélé plus efficace que l'huile minérale et tandis que certains nouveaux SLP ont exercé une activité égale ou supérieure aux SL-Ficoll.

### Exemple 3

Outre l'effet adjuvant, d'autres propriétés sont importantes pour l'évaluation d'un vaccin. Entre autres, la réaction locale est un aspect important, bien qu'une certaine réaction à l'endroit d'injection soit acceptée en général pour certaines espèces d'animaux. La toxicité locale a été testée in vivo en suivant le gonflement des pattes de souris après injection du vaccin. Il a été montré que cette méthode est très sensible.

Des formulations de SLP dans une émulsion de squalane dans l'eau, d'huile de soja dans l'eau, d'huile minérale et d'hexadécane dans l'eau ainsi qu'un témoin sans adjuvant ont été préparés selon la méthode décrite à l'exemple 1.

Des vaccins contenant les formulations avec adjuvant ont été préparés selon la méthode décrite à l'exemple 1.

Des groupes de six souris ont été traités avec 25 µl de vaccin par injection sous-cutanée dans la plante du pieds arrière gauche. Le vaccin comprenait un volume de solution d'antigènes contenant 10µg de MRC-11 et 1 mg d'ovalbumine (OVA) par ml de PBS et un volume d'adjuvant.

L'épaisseur des pattes a été mesurée un jour avant et à plusieurs intervalles après l'injection par un appareil semi-électronique spécialement développé pour ce propos par l'Université d'Etat d'Utrecht aux Pays-Bas. La précision de cet appareil est d'environ 0,02 mm.

Le gonflement a été calculé en soustrayant l'épaisseur de la patte avant le traitement de l'épaisseur de la patte après le traitement et est exprimé en 0,01 mm.

Les résultats de ces expériences sont repris dans le tableau 3.

Tableau 3 : Réactogénicité de différents adjuvants sur des souris

| Expérience I | | | | | | | | | |
|---|---|---|---|---|---|---|---|---|---|
| Adjuvant [mg SLP+µl huile]/ml | gonflement moyen (10⁻² mm) | | | | | | | | |
| | Jours | | | | | | | | |
| | 1 | 2 | 3 | 4 | 7 | 11 | 14 | 18 | 25 |
| SL-Maldex15/S/W [10/100] | 169 | 110 | 89 | 65 | 39 | 16 | 18 | 19 | 17 |
| SL-Cyclodex./S/W [10/100] | 88 | 21 | 8 | 8 | 9 | 3 | 6 | 12 | -1 |
| SL-Inuline/S/W [10/100] | 146 | 145 | 94 | 102 | 44 | 30 | 25 | 26 | 20 |
| SL-Pullulane/S/W [10/100] | 235 | 231 | 137 | 112 | 87 | 74 | 60 | 64 | 42 |
| SL-Maldexl5/soja/W [10/100] | 30 | 10 | 5 | 0 | 2 | 10 | 5 | 5 | 1 |
| SL-Cyclodex./soja/W [10/100] | 2 | 0 | 0 | 0 | 0 | 0 | 4 | 10 | -7 |
| SL-Inuline/soja/W [10/100] | 117 | 64 | 13 | 7 | 7 | 10 | 20 | 18 | 1 |
| SL-Pullulane/soja/W [10/100] | 40 | 23 | 7 | 0 | 0 | 0 | 3 | 10 | -2 |
| SL-Ficoll/S/W [10/100] | 196 | 273 | 299 | 305 | 305 | 237 | 177 | 144 | 97 |
| SL-Ficoll/soja/W [10/100] | 124 | 59 | 24 | 20 | 26 | 17 | 12 | 7 | -5 |
| SL-Ficoll/hexadéc./W [10/100] | 241 | 277 | 242 | 209 | 92 | 35 | 42 | 49 | 14 |
| SL-Ficoll/h. minérale/W [10/100] | 233 | 295 | 300 | 292 | 201 | 165 | 120 | 89 | 75 |
| Contrôle (sans adjuvant) | 2 | 0 | 0 | 0 | 0 | 0 | 4 | -6 | -4 |
| Maldex15 = maltodextrine 15 hexadéc. = hexadécane | | | | | | | | | |
| Cyclodex. = cyclodextrine h. min. = huile minérale | | | | | | | | | |
| soja = huile de soja S = squalane | | | | | | | | | |
| W = eau (water) | | | | | | | | | |

| Expérience II | | | | | | |
|---|---|---|---|---|---|---|
| Adjuvant [mg SLP+µl huile]/ml | gonflement moyen (10⁻² mm) | | | | | |
| | Jours | | | | | |
| | 1 | 2 | 6 | 10 | 17 | 22 |
| SL-Maldexl5/S/W [10/100] | 133 | 95 | 61 | 30 | 35 | 24 |
| SL-Cyclodex./S/W [10/100] | 62 | 54 | 10 | 15 | 17 | 1 |
| SL-Inuline/S/W [10/100] | 176 | 134 | 71 | 51 | 55 | 48 |
| SL-Pullulane/S/W [10/100] | 200 | 199 | 91 | 94 | 77 | 46 |
| SL-Maldex15/soja/W [10/100] | 20 | 10 | 7 | 0 | 0 | 0 |
| SL-Cyclodex./soja/W [10/100] | 19 | 16 | 21 | 16 | 11 | 12 |
| SL-Inuline/soja/W [10/100] | 52 | 38 | 22 | 11 | 24 | 0 |
| SL-Pullulane/soja/W [10/100] | 52 | 38 | 11 | 4 | 15 | 0 |
| SL-Maldexl5/hexadene/W [10/100] | 108 | 65 | 14 | 14 | 22 | 0 |
| SL-Cyclodex./hexadéc./W [10/100] | 107 | 74 | 14 | 10 | 25 | 0 |
| SL-Inuline/hexadéc./W [10/100] | 121 | 114 | 46 | 30 | 22 | 0 |
| SL-Pullulane/hexadéc./W [10/100] | 224 | 213 | 112 | 59 | 40 | 23 |
| Contrôle (sans adjuvant) | 2 | 0 | 0 | 0 | 0 | 0 |
| SL-Ficoll/S/W [10/100] | 73 | 293 | 325 | 239 | 178 | 99 |
| Maldex15 = maltodextrine15 hexadéc. = hexadécane | | | | | | |
| Cyclodex. = cyclodextrine soja = huile de soja | | | | | | |
| S = squalane W = eau | | | | | | |

Les formulations ont provoqué différents degrés de gonflement au point d'injection, ce gonflement qui a été très fort dans certains cas pendant les premiers jours, s'estompait lentement (Tableau 3). Des émulsions comprenant de l'hexadécane ont provoqué, en général, des réactions plus fortes que celles contenant de l'huile de soja. En ce qui concerne les SLP, les émulsions de squalane comprenant du SL-Ficoll ont provoqué des gonflements plus forts qui se sont maintenus pendant plus de trois semaines. Les émulsions contenant du SL-Maltodextrinel5, du SL-Inuline ou du SL-Pullulane provoquaient des réactions de gonflement considérables qui s'estompaient cependant plus rapidement que celles provoquées par le SL-Ficoll et ne persistaient que pendant une à deux semaines.

La réactogénicité du SL-Cyclodextrine inclus dans différentes huiles a été très faible ou même absente. Des émulsions contenant du SL-Cyclodextrine dans du squalane ou de l'hexadécane ont provoqué quelques gonflements pendant un ou deux jours après l'injection tandis que la SL-Cyclodextrine dans de l'huile de soja n'a pas provoqué de réponse visible.

La réactogénicité, c'est-à-dire la toxicité locale, testée sur les souris, montrait des effets prononcés en fonction du type d'huile et du type de polysaccharide. En général, le SL-Ficoll provoquait des réactions plus fortes que les autres SLP testés. Le SL-Ficoll combiné avec soit du squalane soit de l'huile minérale a provoqué une réaction locale très forte et persistante tandis que le SL-Ficoll combiné avec de l'hexadécane et de l'huile de soja n'a donné que des réactions modérées ou plus faibles respectivement (Tableau 2). Des émulsions à base de squalane ont provoqué des réactions significativement plus fortes que celles comprenant de l'hexadécane. L'huile de soja n'a provoqué que des réactions très faibles.

Le caractère hydrophobe de ces huiles est différent. Le squalane, l'huile minérale et l'hexadécane sont des hydrocarbones fortement hydrophobes, tandis que l'huile de soja, étant un mélange de différentes substances, est moins hydrophobe. Le caractère hydrophobe des différentes huiles est illustré par la valeur HLB des détergents nécessaires pour obtenir une émulsion stable dans de l'eau.

Le SL-Pullulane a provoqué des toxicités locales plus fortes que les trois autres SLP. SL-Inuline et SL-Maltodextrinel5 ont provoqué des gonflements locaux considérables tandis que le SL-Cyclodextrine n'a provoqué qu'une réaction notable seulement pendant un ou deux jours.

Parmi les autres caractéristiques, les SLP diffèrent dans leur poids moléculaire qui décroît de SL-Ficoll = à celle SL-Pullulane > SL-Inuline > SL-Maltodextrinel5 > SL-Cyclodextrine. Un parallélisme entre la réactogénicité et le poids moléculaire peut être observé mais d'autres recherches sont requises pour établir une relation directe.

On en a conclu que la réactogénicité dépend du type de polysaccharide et d'huile inclus dans les formulations et que le poids moléculaire du polysaccharide est un facteur important.

### Exemple 4

Des dérivés sulfo-lipidiques de Maltodextrinel5, d'Inuline, de Cyclodextrine et de Pullulane ont été synthétisés et incorporés dans des émulsions d'huile dans de l'eau à base de squalane, d'huile minérale, d'hexadécane et d'huile de soja et la stabilité a été étudiée in vitro.

Les SLP de la présente invention ont été synthétisés selon la méthode décrite à l'exemple 1 et mélangés dans des émulsions huile dans l'eau de squalane, hexadécane et huile de soja et la stabilité de celles-ci a été étudiée in vitro.

Des formulations de SLP dans une émulsion de squalane dans l'eau, d'huile de soja dans l'eau et d'hexadécane dans l'eau ont été préparées selon la méthode décrite à l'exemple 1.

La stabilité des émulsions a été testée en exposant les formulations à température élevée pendant une certaine période de temps. En général, les émulsions sont moins stables à haute température et le test à température élevée a été considéré comme donnant des indications sur le comportement à long terme à température plus faible.

La stabilité des émulsions a été déterminée à 37 °C. Des aliquotes stériles de 5 ml d'émulsion ont été incubés à 37 °C et la formation de gouttelettes d'huile, l'apparition d'une phase huileuse et autres modifications ont été vérifiées chaque jour par inspection des émulsions à l'oeil nu.

Les SLP avec un rapport S/L d'environ 0,1/1,0 ont été incorporés dans des émulsions de type huile dans eau à base de squalane, de l'hexadécane et d'huile de soja.

Des aliquotes stériles comprenant 0,01 % (p/v) de thimérosal (SIGMA) ont été incubés à 37 °C et l'état des émulsions a été établie à différents intervalles de temps par inspection à l'oeil nu.

Les résultats de ces expériences sont repris dans le Tableau 4.

Tableau 4 : Stabilité de différentes émulsions de différentes huiles comprenant des SLP en une concentration finale de 1 % (p/v) SLP, 2 % (v/v) TWEEN 80, 10 % (v/v) d'huile dans du PBS.

| Adjuvant [mg SLP+µl huile]/ml | Stabilité à 37 °C au jour : | | | | | | | | | | |
|---|---|---|---|---|---|---|---|---|---|---|---|
| | 1 | 2 | 7 | 10 | 14 | 21 | 28 | 35 | 46 | 53 | 115 |
| SL-Maldexl5/S/W [10/100] | + | + | + | + | ± | + | + | + | + | + | ±/+ |
| SL-Cyclodex./S/W [10/100] | + | + | + | + | + | + | + | + | + | + | ±/+ |
| SL-Inuline/S/W [10/100] | + | + | + | + | + | + | ± | ± | + | + | + |
| SL-Pullulane/S/W [10/100] | + | + | + | + | + | + | + | + | ± | - | -- |
| SL-Maldex15/soja/W [10/100] | + | + | + | + | + | + | + | + | + | + | + |
| SL-Cyclodex./soja/W [10/100] | + | + | + | + | + | + | + | + | + | + | + |
| SL-Inuline/soja/W [10/100] | + | + | + | + | + | + | + | + | + | + | + |
| SL-Pullulane/soja/W [10/100] | + | + | + | + | + | + | + | + | + | + | - |
| SL-Maldexl5/hexadéc./W [10/100] | + | + | + | + | + | + | + | + | + | + | + |
| SL-Cyclodex./hexadéc./ W [10/100] | + | | + | + | + | + | + | + | + | + | ± |
| SL-Inuline/hexadéc./W [10/100] | + | + | + | + | + | + | + | + | + | + | ± |
| SL-Pullulane/hexadéc./W [10/100] | + | + | + | + | + | + | + | + | + | + | - |
| Maldex15 = Maltodextrine15 | | | | | | | | | | | |
| Cyclodex. = cyclodextrine | | | | | | | | | | | |
| soja = huile de soja | | | | | | | | | | | |
| hexadéc. = hexadécane | | | | | | | | | | | |
| S = squalane | | | | | | | | | | | |
| W = eau (water) | | | | | | | | | | | |
| -- = deux phases; phase huileuse et phase aqueuse transparente | | | | | | | | | | | |
| - = deux phases; phase huileuse et phase aqueuse blanche | | | | | | | | | | | |
| ± = petites gouttelettes d'huile sur la phase aqueuse visible à l'oeil | | | | | | | | | | | |
| nu | | | | | | | | | | | |
| + = émulsion homogène blanche, pas de gouttelettes d'huiles | | | | | | | | | | | |
| visibles à l'oeil nu | | | | | | | | | | | |
| ±/+ = entre "±" et "+" | | | | | | | | | | | |

Les nouveaux SLP (SL-Pullulane, SL-Inuline, SL-Maltodextrinel5, et SL-Cyclodextrine) incorporés dans ces émulsions d'huile de squalane, d'hexadécane ou d'huile de soja sont d'une stabilité remarquable. Les émulsions de SL-Inuline, SL-Cyclodextrine et SL-Maltodextrinel5 dans du squalane sont restées stables plus de 53 jours à 37 °C. Ceci est considéré comme étant une amélioration importante en comparaison avec les émulsions de SL-Ficoll/squalane/eau. En dépit de la valeur prédictive limitée du test de stabilité à 37 °C, celle-ci montre une résistance accrue contre les facteurs de déstabilisation.

Toutes les émulsions, à l'exception de SL-Pullulane/squalane/eau, sont restées stables pendant au moins 53 jours à 37 °C. Après 115 jours, la plupart des émulsions comprenant SL-Maltodextrinel5, SL-Cyclodextrine et SL-Inuline sont restées stables, bien que des émulsions avec du squalane et de l'hexadécane montraient quelques gouttelettes d'huile sur l'émulsion.

## Revendications

1. Adjuvant pour vaccins, comprenant un polysaccharide sulfolipidique hydrophobe combiné à un constituant formant une interface, dans lequel le polysaccharide sulfolipidique est choisi dans le groupe constitué par les cyclodextrines, la maltodextrine, l'inuline et le pullulane, les groupes lipidiques comprennent 4 à 22 atomes de carbone et le polysaccharide sulfolipidique contient, en moyenne, au moins 0,01 et pas plus de 1,0 groupe sulfate par monosaccharide, en moyenne, au moins 0,01 et pas plus de 2,0 groupes lipidiques par monosaccharide, et le rapport des groupes sulfate aux groupes lipidiques est un rapport de 0,01 à 2 groupes sulfate par groupe lipidique, tout en maintenant son caractère hydrophobe.

2. Adjuvant suivant la revendication 1, caractérisé en ce que le polysaccharide sulfolipidique est une cyclodextrine.

3. Adjuvant suivant la revendication 1, caractérisé en ce que le polysaccharide sulfolipidique contient, en moyenne, au moins 0,12 groupe sulfate par monosaccharide, tout en maintenant son caractère hydrophobe.

4. Adjuvant suivant la revendication 1, caractérisé en ce que le polysaccharide sulfolipidique ne contient, en moyenne, pas plus de 0,23 groupe sulfate par monosaccharide, tout en maintenant son caractère hydrophobe.

5. Adjuvant suivant la revendication 1, caractérisé en ce que le polysaccharide sulfolipidique est de la maltodextrine et contient, en moyenne 0,23 groupe sulfate par monosaccharide, tout en maintenant son caractère hydrophobe.

6. Adjuvant suivant la revendication 1, caractérisé en ce que le polysaccharide sulfolipidique est de la cyclodextrine et contient, en moyenne, 0,20 groupe sulfate par monosaccharide, tout en maintenant son caractère hydrophobe.

7. Adjuvant suivant la revendication 1, caractérisé en ce que le polysaccharide sulfolipidique est de l'inuline et contient, en moyenne, 0,19 groupe sulfate par monosaccharide, tout en maintenant son caractère hydrophobe.

8. Adjuvant suivant la revendication 1, caractérisé en ce que le polysaccharide sulfolipidique est du pullulane et contient, en moyenne, 0,16 groupe sulfate par monosaccharide, tout en maintenant son caractère hydrophobe.

9. Adjuvant suivant la revendication 1, caractérisé en ce que le polysaccharide sulfolipidique contient, en moyenne, au moins 1,05 groupe lipidique par monosaccharide, tout en maintenant son caractère hydrophobe.

10. Adjuvant suivant la revendication 1, caractérisé en ce que le polysaccharide sulfolipidique, en moyenne, ne contient pas plus de 1,29 groupe lipidique par monosaccharide, tout en maintenant son caractère hydrophobe.

11. Adjuvant suivant la revendication 1, caractérisé en ce que le polysaccharide sulfolipidique est de la maltodextrine contenant, en moyenne, 1,29 groupe lipidique par monosaccharide, tout en maintenant son caractère hydrophobe.

12. Adjuvant suivant la revendication 1, caractérisé en ce que le polysaccharide sulfolipidique est de la cyclodextrine contenant, en moyenne, 1,05 groupe lipidique par monosaccharide, tout en maintenant son caractère hydrophobe.

13. Adjuvant suivant la revendication 1, caractérisé en ce que le polysaccharide sulfolipidique est de l'inuline contenant, en moyenne 1,24 groupe lipidique par monosaccharide, tout en maintenant son caractère hydrophobe.

14. Adjuvant suivant la revendication 1, caractérisé en ce que le polysaccharide sulfolipidique est du pullulane contenant, en moyenne, 1,24 groupe lipidique par monosaccharide, tout en maintenant son caractère hydrophobe.

15. Adjuvant suivant la revendication 1, caractérisé en ce que le rapport des groupes sulfate aux groupes lipidiques est un rapport de 0,10-0,19 groupe sulfate par groupe lipidique, tout en maintenant le caractère hydrophobe du composé.

16. Adjuvant suivant la revendication 1, caractérisé en ce que le polysaccharide sulfolipidique est de la maltodextrine contenant un rapport des groupes sulfate aux groupes lipidiques d'approximativement 0,18 groupe sulfate par groupe lipidique, tout en maintenant son caractère hydrophobe.

17. Adjuvant suivant la revendication 1, caractérisé en ce que le polysaccharide sulfolipidique est de la cyclodextrine contenant un rapport des groupes sulfate aux groupes lipidiques d'approximativement 0,19 groupe sulfate par groupe lipidique, tout en maintenant son caractère hydrophobe.

18. Adjuvant suivant la revendication 1, caractérisé en ce que le polysaccharide sulfolipidique est de l'inuline contenant un rapport des groupes sulfate aux groupes lipidiques d'approximativement 0,15 groupe sulfate par groupe lipidique, tout en maintenant son caractère hydrophobe.

19. Adjuvant suivant la revendication 1, caractérisé en ce que le polysaccharide sulfolipidique est du pullulane contenant un rapport des groupes sulfate aux groupes lipidiques d'approximativement 0,13 groupe sulfate par groupe lipidique, tout en maintenant son caractère hydrophobe.

20. Adjuvant suivant la revendication 1, caractérisé en ce que le constituant formant une interface est choisi dans le groupe consistant en un liquide non miscible à l'eau et une substance solide qui est insoluble dans la phase aqueuse.

21. Adjuvant suivant la revendication 1, caractérisé en ce que le liquide non miscible à l'eau est choisi dans le groupe consistant en squalane, huile de soja, une huile minérale et hexadécane.

22. Utilisation d'un polysaccharide sulfolipidique suivant l'une quelconque des revendications 1 à 21 comme adjuvant dans des vaccins.

23. Procédé pour la préparation d'un vaccin en émulsion, caractérisé en ce que l'on émulsionne une solution aqueuse d'un antigène, un polysaccharide sulfolipidique suivant l'une quelconque des revendications 1 à 21, un constituant formant une interface et un émulsifiant.

24. Vaccin comprenant une quantité immunogène d'un antigène et un adjuvant comprenant un polysaccharide sulfolipidique suivant l'une quelconque des revendications 1 à 21 et un constituant formant une interface.

## Patentansprüche

1. Adjuvans für Impfstoffe, umfassend ein hydrophobes Sulfolipid-Polysaccharid, das mit einer Komponente verbunden ist, die eine Grenzfläche bildet, in dem das Sulfolipid-Polysaccharid ausgewählt ist aus der Gruppe bestehend aus den Zyklodextrinen, dem Maltodextrin, dem Inulin und dem Pullulan, wobei die Lipidgruppen 4 bis 22 Kohlenstoffatome umfassen und die Sulfolipid-Polysaccharide durchschnittlich zumindest 0,01 und nicht mehr als 1,0 Sulfatgruppen pro Monosaccharid, durchschnittlich zumindest 0,01 und nicht mehr als 2,0 Lipidgruppen pro Monosaccharid aufweist und das Verhältnis der Sulfatgruppen zu den Lipidgruppen ein Verhältnis von 0,01 bis 2 Sulfatgruppen pro Lipidgruppe ist, wobei dessen hydrophobe Eigenschaft aufrechterhalten wird.

2. Adjuvans nach Anspruch 1, dadurch gekennzeichnet, dass das Sulfolipid-Polysaccharid ein Zyklodextrin ist.

3. Adjuvans nach Anspruch 1, dadurch gekennzeichnet, dass das Sulfolipid-Polysaccharid durchschnittlich zumindest 0,12 Sulfatgruppen pro Monosaccharid aufweist, wobei dessen hydrophobe Eigenschaft aufrechterhalten wird.

4. Adjuvans nach Anspruch 1, dadurch gekennzeichnet, dass das Sulfolipid-Polysaccharid durchschnittlich nicht mehr als 0,23 Sulfatgruppen pro Monosaccharid aufweist, wobei dessen hydrophobe Eigenschaft aufrechterhalten wird.

5. Adjuvans nach Anspruch 1, dadurch gekennzeichnet, dass das Sulfolipid-Polysaccharid Maltodextrin ist und durchschnittlich 0,23 Sulfatgruppen pro Monosaccharid aufweist, wobei dessen hydrophobe Eigenschaft aufrechterhalten wird.

6. Adjuvans nach Anspruch 1, dadurch gekennzeichnet, dass das Sulfolipid-Polysaccharid Zyklodextrin ist und durchschnittlich 0,20 Sulfatgruppen pro Monosaccharid aufweist, wobei dessen hydrophobe Eigenschaft aufrechterhalten wird.

7. Adjuvans nach Anspruch 1, dadurch gekennzeichnet, dass das Sulfolipid-Polysaccharid Inulin ist und durchschnittlich 0,19 Sulfatgruppen pro Monosaccharid aufweist, wobei dessen hydrophobe Eigenschaft aufrechterhalten wird.

8. Adjuvans nach Anspruch 1, dadurch gekennzeichnet, dass das Sulfolipid-Polysaccharid Pullulan ist und durchschnittlich 0,16 Sulfatgruppen pro Monosaccharid aufweist, wobei dessen hydrophobe Eigenschaft aufrechterhalten wird.

9. Adjuvans nach Anspruch 1, dadurch gekennzeichnet, dass das Sulfolipid-Polysaccharid durchschnittlich zumindest 1,05 Lipidgruppen pro Monosaccharid aufweist, wobei dessen hydrophobe Eigenschaft aufrechterhalten wird.

10. Adjuvans nach Anspruch 1, dadurch gekennzeichnet, dass das Sulfolipid-Polysaccharid durchschnittlich nicht mehr als 1,29 Lipidgruppen pro Monosaccharid aufweist, wobei dessen hydrophobe Eigenschaft aufrechterhalten wird.

11. Adjuvans nach Anspruch 1, dadurch gekennzeichnet, dass das Sulfolipid-Polysaccharid Maltodextrin ist, aufweisend durchschnittlich 1,29 Lipidgruppen pro Monosaccharid, wobei dessen hydrophobe Eigenschaft aufrechterhalten wird.

12. Adjuvans nach Anspruch 1, dadurch gekennzeichnet, dass das Sulfolipid-Polysaccharid Zyklodextrin ist, aufweisend durchschnittlich 1,05 Lipidgruppen pro Monosaccharid, wobei dessen hydrophobe Eigenschaft aufrechterhalten wird.

13. Adjuvans nach Anspruch 1, dadurch gekennzeichnet, dass das Sulfolipid-Polysaccharid Inulin ist, aufweisend durchschnittlich 1,24 Lipidgruppen pro Monosaccharid, wobei dessen hydrophobe Eigenschaft aufrechterhalten wird.

14. Adjuvans nach Anspruch 1, dadurch gekennzeichnet, dass das Sulfolipid-Polysaccharid Pullulan ist, aufweisend durchschnittlich 1,24 Lipidgruppen pro Monosaccharid, wobei dessen hydrophobe Eigenschaft aufrechterhalten wird.

15. Adjuvans nach Anspruch 1, dadurch gekennzeichnet, dass das Verhältnis der Sulfatgruppen zu den Lipidgruppen ein Verhältnis von 0,10 bis 0,19 Sulfatgruppen pro Lipidgruppe ist, wobei die hydrophobe Eigenschaft der Verbindung aufrechterhalten wird.

16. Adjuvans nach Anspruch 1, dadurch gekennzeichnet, dass das Sulfolipid-Polysaccharid Maltodextrin ist, aufweisend ein Verhältnis der Sulfatgruppen zu den Lipidgruppen von ungefähr 0,18 Sulfatgruppen pro Lipidgruppe, wobei dessen hydrophobe Eigenschaft aufrechterhalten wird.

17. Adjuvans nach Anspruch 1, dadurch gekennzeichnet, dass das Sulfolipid-Polysaccharid Zyklodextrin ist, aufweisend ein Verhältnis der Sulfatgruppen zu den Lipidgruppen von ungefähr 0,19 Sulfatgruppen pro Lipidgruppe, wobei dessen hydrophobe Eigenschaft aufrechterhalten wird.

18. Adjuvans nach Anspruch 1, dadurch gekennzeichnet, dass das Sulfolipid-Polysaccharid Inulin ist, aufweisend ein Verhältnis der Sulfatgruppen zu den Lipidgruppen von ungefähr 0,15 Sulfatgruppen pro Lipidgruppe, wobei dessen hydrophobe Eigenschaft aufrechterhalten wird.

19. Adjuvans nach Anspruch 1, dadurch gekennzeichnet, dass das Sulfolipid-Polysaccharid Pullulan ist, aufweisend ein Verhältnis der Sulfatgruppen zu den Lipidgruppen von ungefähr 0,13 Sulfatgruppen pro Lipidgruppe, wobei dessen hydrophobe Eigenschaft aufrechterhalten wird.

20. Adjuvans nach Anspruch 1, dadurch gekennzeichnet, dass die die Grenzfläche bildende Komponente ausgewählt ist aus der Gruppe bestehend aus einer mit Wasser nicht mischbaren Flüssigkeit und einem Feststoff, der in der wässerigen Phase nicht lösbar ist.

21. Adjuvans nach Anspruch 1, dadurch gekennzeichnet, dass die mit Wasser nicht mischbare Flüssigkeit ausgewählt ist aus der Gruppe bestehend aus Squalan, Sojaöl, ein Mineralöl und Hexadecan.

22. Verwendung eines Sulfolipid-Polysaccharids gemäß einem der Ansprüche 1 bis 21 als Adjuvans in Impfstoffen.

23. Verfahren zur Herstellung eines Impfstoffes in Emulsion, dadurch gekennzeichnet, dass eine wässerige Lösung eines Antigens, ein Sulfolipid-Polysaccharid nach einem der Ansprüche 1 bis 21, eine eine Grenzfläche bildende Komponente und ein Emulgator emulgiert werden.

24. Impfstoff, umfassend eine immunogene Menge eines Antigens und ein Adjuvans, umfassend ein Sulfolipid-Polysaccharid nach einem der Ansprüche 1 bis 21 und eine eine Grenzfläche bildende Komponente.

## Claims

1. Vaccine adjuvant comprising a hydrophobic sulpholipid polysaccharide combined with an interface-forming constituent, in which the sulpholipid polysaccharide is chosen from the group consisting of cyclodextrins, maltodextrin, inulin and pullulan, the lipid groups comprising 4 to 22 carbon atoms and the sulpholipid polysaccharide contains, on average, at least 0.01 and not more than 1.0 sulphate group per monosaccharide, on average, at least 0.01 and not more than 2.0 lipid groups per monosaccharide, and the ratio of the sulphate groups to the lipid groups is a ratio of 0.01 to 2 sulphate groups per lipid group, while maintaining its hydrophobic character.

2. Adjuvant according to Claim 1, characterized in that the sulpholipid polysaccharide is a cyclodextrin.

3. Adjuvant according to Claim 1, characterized in that the sulpholipid polysaccharide contains, on average, at least 0.12 sulphate group per monosaccharide, while maintaining its hydrophobic character.

4. Adjuvant according to Claim 1, characterized in that the sulpholipid polysaccharide contains, on average, not more than 0.23 sulphate group per monosaccharide, while maintaining its hydrophobic character.

5. Adjuvant according to Claim 1, characterized in that the sulpholipid polysaccharide is maltodextrin and contains, on average, 0.23 sulphate group per monosaccharide, while maintaining its hydrophobic character.

6. Adjuvant according to Claim 1, characterized in that the sulpholipid polysaccharide is cyclodextrin and contains, on average, 0.20 sulphate group per monosaccharide, while maintaining its hydrophobic character.

7. Adjuvant according to Claim 1, characterized in that the sulpholipid polysaccharide is inulin and contains, on average, 0.19 sulphate group per monosaccharide, while maintaining its hydrophobic character.

8. Adjuvant according to Claim 1, characterized in that the sulpholipid polysaccharide is pullulan and contains, on average, 0.16 sulphate group per monosaccharide, while maintaining its hydrophobic character.

9. Adjuvant according to Claim 1, characterized in that the sulpholipid polysaccharide contains, on average, at least 1.05 lipid groups per monosaccharide, while maintaining its hydrophobic character.

10. Adjuvant according to Claim 1, characterized in that the sulpholipid polysaccharide, on average, contains not more than 1.29 lipid groups per monosaccharide, while maintaining its hydrophobic character.

11. Adjuvant according to Claim 1, characterized in that the sulpholipid polysaccharide is maltodextrin containing, on average, 1.29 lipid groups per monosaccharide, while maintaining its hydrophobic character.

12. Adjuvant according to Claim 1, characterized in that the sulpholipid polysaccharide is cyclodextrin containing, on average, 1.05 lipid groups per monosaccharide, while maintaining its hydrophobic character.

13. Adjuvant according to Claim 1, characterized in that the sulpholipid polysaccharide is inulin containing, on average, 1.24 lipid groups per monosaccharide, while maintaining its hydrophobic character.

14. Adjuvant according to Claim 1, characterized in that the sulpholipid polysaccharide is pullulan containing, on average, 1.24 lipid groups per monosaccharide, while maintaining its hydrophobic character.

15. Adjuvant according to Claim 1, characterized in that the ratio of the sulphate groups to the lipid groups is a ratio of 0.10-0.19 sulphate group per lipid group, while maintaining the hydrophobic character of the compound.

16. Adjuvant according to Claim 1, characterized in that the sulpholipid polysaccharide is maltodextrin containing a ratio of sulphate groups to lipid groups of approximately 0.18 sulphate group per lipid group, while maintaining its hydrophobic character.

17. Adjuvant according to Claim 1, characterized in that the sulpholipid polysaccharide is cyclodextrin containing a ratio of the sulphate groups to the lipid groups of approximately 0.19 sulphate group per lipid group, while maintaining its hydrophobic character.

18. Adjuvant according to Claim 1, characterized in that the sulpholipid polysaccharide is inulin containing a ratio of the sulphate groups to the lipid groups of approximately 0.15 sulphate group per lipid group, while maintaining its hydrophobic character.

19. Adjuvant according to Claim 1, characterized in that the sulpholipid polysaccharide is pullulan containing a ratio of the sulphate groups to the lipid groups of approximately 0.13 sulphate group per lipid group, while maintaining its hydrophobic character.

20. Adjuvant according to Claim 1, characterized in that the interface-forming constituent is chosen from the group consisting of a liquid which is immiscible with water and a solid substance which is insoluble in the aqueous phase.

21. Adjuvant according to Claim 1, characterized in that the liquid which is immiscible with water is chosen from the group consisting of squalane, soyabean oil, a mineral oil and hexadecane.

22. Use of a sulpholipid polysaccharide according to any one of Claims 1 to 21 as adjuvant in vaccines.

23. Method of preparing a vaccine in emulsion, characterized in that an aqueous solution of an antigen, a sulpholipid polysaccharide according to any one of Claims 1 to 21, an interface-forming constituent and an emulsifier are emulsified.

24. Vaccine comprising an immunogenic quantity of an antigen and an adjuvant comprising a sulpholipid polysaccharide according to any one of Claims 1 to 21 and an interface-forming constituent.
